# EUROPEAN PATENT SPECIFICATION

(11) **EP 3 123 958 B1**
(45) Date of publication and mention of the grant of the patent: **27.02.2019**
(21) Application number: 16181395.1
(22) Date of filing: 27.07.2016
(51) Int. Cl.: A61B 17/115

(54) **LOADING UNIT LOCKING COLLAR**
LADEEINHEITVERRIEGELUNGSRING
COLLIER DE VERROUILLAGE D'UNITÉ DE CHARGEMENT

(30) Priority: 28.07.2015 US 201514810811
(43) Date of publication of application: 01.02.2017
(73) Proprietor: Covidien LP, Mansfield, MA 02048 (US)
(72) Inventor: Penna, Christopher, Guilford, CT 06437 (US); Sgroi, Anthony, Wallingford, CT 06492 (US)
(74) Representative: Soames, Candida Jane

(56) References cited:
- WO-A1-2014/139327
- WO-A2-98/05261
- US-A1- 2004 059 227
- US-A1- 2008 179 375
- US-A1- 2011 276 036
- US-A1- 2013 123 705

## Description

### BACKGROUND

### 1. Technical Field

The present disclosure relates generally to surgical stapling instruments. More specifically, the present disclosure relates to circular surgical stapling instruments including replaceable loading units.

### 2. Background of Related Art

Surgical stapling devices configured to join tissue portions during a surgical procedure are well known. These devices include linear end effectors which are oriented parallel or transverse to a longitudinal axis of the device as well as circular end effectors. Typically, linear stapling devices include a disposable loading unit or a replaceable cartridge that allows the stapling device to be used multiple times. However, conventional circular stapling devices include a cartridge or shell assembly that is fixedly attached to the device such that the device must be disposed of after a single use. Some circular stapling devices include a cartridge or shell assembly that is replaceable. Document US2013/123705 discloses a loading unit and locking collar according to the preamble of claim 1.

A need exists in the art for a simple, inexpensive device for releasably securing a cartridge or shell assembly to a circular stapling device to facilitate reuse of the stapling device.

### SUMMARY

The invention is disclosed in the appended set of claims. In the present invention, a loading unit and locking collar assembly includes a shell assembly and a locking collar. The shell assembly has a proximal end portion for receiving a distal end portion of a surgical instrument. The proximal end portion includes an annular ring that has an outer surface and an inner surface. The outer surface of the annular ring defines an annular groove with the proximal end portion. The annual ring defines a lock opening therethrough. The locking collar is releasably disposed within the annular groove and includes an annular body. The annular body of the locking collar has a pair of release surfaces and a lock. The lock extends radially inward from the annular body. The pair of release surfaces is configured to transition the locking collar from a locked configuration to an unlocked configuration. In the locked configuration, the lock extends through the lock opening to penetrate the inner surface of the annular ring and in the unlocked configuration the lock is positioned radially outward from the inner surface of the annular ring.

In the invention, each of the release surfaces is urged towards the other release surface to transition the locking collar from the locked configuration to the unlocked configuration. Each of the release surfaces is compressed radially inward to transition the locking collar towards the unlocked configuration. Alternatively, each of the release surfaces may be urged about the circumference of the locking collar in a plane transverse to a longitudinal axis of the shell assembly to transition the locking collar towards the unlocked configuration.

In some aspects, the locking collar defines an elliptical cross-section in a plane transverse to a longitudinal axis of the shell assembly in which the width in the unlocked configuration is less than the width in the locked configuration. The width may be defined between the pair of release surfaces. Alternatively, the locking collar may define an elliptical cross-section in a plane transverse to a longitudinal axis of the shell assembly in which the width is less than the height.

In certain aspects, the locking collar defines a circular cross-section in a plane transverse to a longitudinal axis of the shell assembly in the locked configuration. The locking collar may define an elliptical cross-section in a plane transvers to a longitudinal axis of the shell assembly in which the width is less than the height in the unlocked configuration. The height may be defined between the pair of release surfaces and the lock.

In particular aspects, the locking collar is biased towards the locked configuration. The annular body of the locking collar may split to from the first and second release surfaces. The first release surface may extend in a first direction about the circumference of the annular body and the second release surface may extend in a second direction opposite the first direction about the circumference of the annular body. The first and second release surfaces may each include an end that defines an engagement window between the end and the annular body. The locking collar may be configured to provide indicia when the locking collar transitions from the unlocked configuration to the locked configuration. The indicia may be audible. The shell assembly may be configured to fire staples through tissue.

In another aspect of the present disclosure, a surgical system includes a surgical instrument, a loading unit, and a locking collar. The surgical instrument includes a distal end. A loading unit includes a shell assembly that has a proximal end portion positioned over the distal end of the surgical instrument. The proximal end portion includes an annular ring that has an outer surface and an inner surface. The outer surface of the annular ring defines an annular groove with the proximal end portion. The annular ring defines a lock opening therethrough. The locking collar is releasably disposed within the annular groove of the loading unit and includes an annular body that has a pair of release surfaces and a lock. The lock extends radially inward from the annular body. The pair of release surfaces is configured to transition the locking collar from a locked configuration to an unlocked configuration. In the locked configuration the lock extends through the lock opening to penetrate the inner surface of the annular ring and in the unlocked configuration the lock is positioned radially outward from the inner surface of the annular ring.

In aspects, the distal end of the surgical instrument defines a window. The lock may extend into the window in the locked configuration and may be positioned outside the window in the unlocked configuration. The locking collar may secure the loading unit to the distal end of the surgical instrument in the locked configuration and the locking collar may allow the loading unit to be removed from the surgical instrument in the unlocked configuration. The lock may include a proximal step, a distal step, and an angled surface between the proximal and distal steps. The angled surface may be configured to slide over the distal end portion of the surgical instrument to move the lock radially outward until the lock is positioned within windows defined in the distal end of the surgical instrument.

In another aspect of the present disclosure, a loading unit and locking collar assembly includes a shell assembly and a locking collar. The shell assembly has a proximally extending annular ring that defines a cylindrical opening. The annular ring defines a lock opening that passes through the annular ring. The shell assembly defines a first retention slot that is adjacent the annular ring on an opposite side of the annular ring from the lock opening. The locking collar is positioned about the annular ring. The locking collar has a body, a lock, and a first retention tab. The body includes at least one release surface. The lock extends from an inner surface of the body spaced from the at least one release surface. The locking collar has a locked configuration and an unlocked configuration. In the locked configuration, the lock extends through the lock opening into the cylindrical opening. In the unlocked configuration, the lock is disposed outwardly of the cylindrical opening. The first retention tab is received within the first retention slot to prevent a side of the locking collar opposite the lock from moving outwardly in relation to the annular ring when the at least one release surface is pressed inwardly towards the annular ring.

In the invention, the at least one release surface includes two release surfaces positioned on opposite sides of the body. The two release surfaces are moveable inwardly towards one another to transition the locking collar to the unlocked configuration. The annular ring may define a relief groove adjacent each of the two release surfaces. The release grooves may allow inward movement of the body of the locking collar in the area of the release surfaces. The body may be resilient and the resilience of the body may urge the locking collar towards the locked configuration.

In some aspects, the assembly includes a retention ring that is positioned about the annular ring proximal of the locking collar. The retention ring may define a second retention slot. The locking collar may include a second retention tab that is received within the second retention slot to prevent the side of the locking collar opposite the lock from moving outwardly when the at least one release surface is pressed inwardly towards the annular ring. The first and second retention tabs may be axially aligned with one another.

In particular aspects, the annular ring defines a retention groove that extends in a direction parallel to a longitudinal axis of the shell assembly. The first and second retention tabs extend from opposite sides of a retention rib that extends inwardly from an inner surface of the body. The retention rib may be disposed in the retention groove. The shell assembly may include a cover that extends distally over the retention groove to define the first retention slot. The body of the locking collar may define a cutout positioned adjacent the first retention tab. The cutout may be sized to receive the cover of the shell assembly.

In certain aspects, the annular ring defines retention openings that pass through the annular ring. The retention ring may include locking tabs that extend from an inner surface of the retention ring which are each received in a respective one of the retention openings to fix the retention ring to the annular ring. The retention openings may be unequally spaced about the annular ring. The locking tabs may define a wedge shape with an angled surface of the wedge facing distally. A proximal end of the annular ring may define chamfers that are each axially aligned with each of the retention openings and/or the lock opening.

In another aspect of the present disclosure, a surgical system includes a surgical instrument, a shell assembly, and a locking collar. The surgical instrument includes a distal end that defines a lock window through the distal end. The shell assembly has a proximally extending annular ring that defines a cylindrical opening. The annular ring defines a lock opening that passes through the annular ring. The shell assembly defines a first retention slot adjacent the annular ring on an opposite side of the annular ring from the lock opening. The distal end of the surgical instrument is received within the cylindrical opening with the lock opening radially aligned with the lock window. The locking collar is positioned about the annular ring. The locking collar has a body, a lock, and a first retention tab. The body includes a release surface. The lock extends from an inner surface of the body spaced apart from the release surface. The locking collar has a locked configuration and an unlocked configuration. In the locked configuration, the lock extends into the lock windows to secure the shell assembly to the distal end of the surgical instrument. In the unlocked configuration, the lock is disengaged from the lock window such that the shell assembly is detachable from the distal end of the surgical instrument. The first retention tab is received within the first retention slot to prevent a side of the locking collar opposite the lock from moving outwardly in relation to the annular ring when the release surface is pressed inwardly towards the annular ring.

In another aspect of the present disclosure, a method of using a loading unit includes aligning a locking collar with a shell assembly and sliding the loading unit distally over a proximally extending annular ring of the shell assembly. A lock of the locking collar engages the annular ring such that the lock is moved outwardly towards an unlocked configuration of the locking collar until the lock is aligned with a lock opening defined through the annular ring. Resilience of a body of the lock collar urges the locking collar towards a locked configuration of the locking collar to move the lock through the lock opening. A retention tab of the locking collar is positioned on an opposite side of the locking collar from the lock and slides into a retention slot defined by the shell assembly to prevent the opposite side of the locking collar from moving outwardly.

In aspects, the method includes attaching the loading unit to a distal end of a surgical instrument by aligning the loading unit with the distal end of the surgical instrument and sliding the loading unit over the distal end of the surgical instrument such that the lock of the locking engages the distal end of the surgical instrument. The distal end may move the lock outwardly to urge the locking collar towards the unlocked configuration until the lock is aligned with a locking window defined in the distal end of the surgical instrument. The lock may be urged through the locking window by resilience of the body of the locking collar to secure the loading unit to the distal end of the surgical instrument.

In some aspects, the method includes actuating a shell assembly of the loading unit with the surgical instrument after the loading unit is attached to the distal end of the surgical instrument. The method may include detaching the loading unit from the distal end of the surgical instrument after actuating the shell assembly by moving a release surface of the locking collar inwardly to move the lock outwardly and sliding the loading unit off of the distal end of the surgical instrument. Moving the release surface of the locking collar inwardly may disengage the lock from the locking window of the distal end of the surgical instrument. The retention tab of the locking collar may engage the shell assembly to prevent the side of the locking collar opposite the lock from moving outwardly in response to moving the release surface of the locking collar inwardly.

Further, to the extent consistent, any of the aspects described herein may be used in conjunction with any or all of the other aspects described herein.

### BRIEF DESCRIPTION OF THE DRAWINGS

Various aspects of the present disclosure are described hereinbelow with reference to the drawings, which are incorporated in and constitute a part of this specification, wherein:
FIG. 1A is a perspective view of a circular stapling surgical instrument in accordance with the present disclosure with a loading unit releasably coupled to a distal end of the surgical instrument;
FIG. IB is a perspective view of another circular stapling adapter in accordance with the present disclosure with the loading unit of FIG. 1A releasably coupled to a distal end of the surgical instrument;
FIG. 2A is a perspective view of the adapter of FIG. 1A with the loading unit decoupled from the surgical instrument;
FIG. 2B is a perspective view of the loading unit of FIG. 1A with the locking collar separated therefrom;
FIG. 3 is an enlarged side view of the distal end of the surgical instrument and loading unit of FIG. 1A;
FIG. 4 is a side cross-sectional view taken along the longitudinal axis of FIG. 3;
FIG. 5 is an enlarged view of the indicated area of detail of FIG. 4;
FIG. 6 is a partial cross-sectional view taken along the section line 6-6 of FIG. 4 illustrating showing the distal end of the surgical instrument and the locking collar in the locked configuration;
FIG. 7 is a partial cross-sectional view similar to FIG. 6 with the distal end of the adapter and the locking collar in the unlocked configuration with a locked configuration of the locking collar shown in dashed lines;
FIG. 8 is an enlarged side cross-sectional view similar to FIG. 5 with the locking collar in the unlocked configuration;
FIG. 9 is a perspective view of another locking collar in accordance with the present disclosure;
FIG. 10 is a front view of the locking collar of FIG. 9 in a locked configuration;
FIG. 11 is a cross-sectional view taken along the section line 11-11 of FIG. 10;
FIG 12 is an end view of the locking collar of FIG. 9 in an unlocked configuration with a locked configuration of the locking collar shown in dashed lines;
FIG. 13 is a perspective view of another loading unit provided in accordance with the present disclosure;
FIGS. 14A and 14B are exploded perspective views of the loading unit of FIG. 13;
FIG. 15 is a cross-sectional view taken along the section line 15-15 of FIG. 13;
FIG. 16 is an enlarged view of the indicated area of detail of FIG. 15;
FIG. 17 is a cross-sectional view taken along the section line 17-17 of FIG. 13;
FIG. 18 is a rear view of the loading unit of FIG. 13 with the locking collar in a locked configuration; and
FIG. 19 is a rear view of the loading unit of FIG. 13 with the locking collar in an unlocked configuration.

### DETAILED DESCRIPTION OF EMBODIMENTS

Embodiments of the present disclosure are now described in detail with reference to the drawings in which like reference numerals designate identical or corresponding elements in each of the several views. As used herein, the term "clinician" refers to a doctor, a nurse, or any other care provider and may include support personnel. Throughout this description, the term "proximal" refers to the portion of the device or component thereof that is closest to the clinician and the term "distal" refers to the portion of the device or component thereof that is farthest from the clinician.

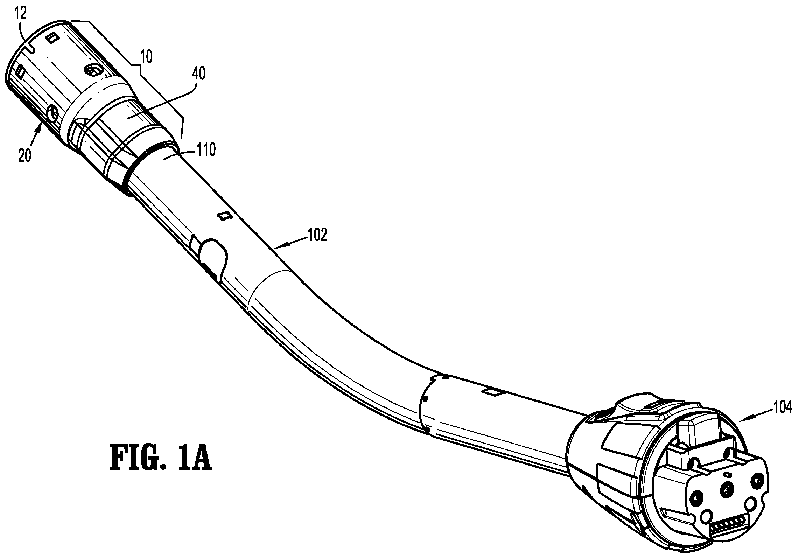

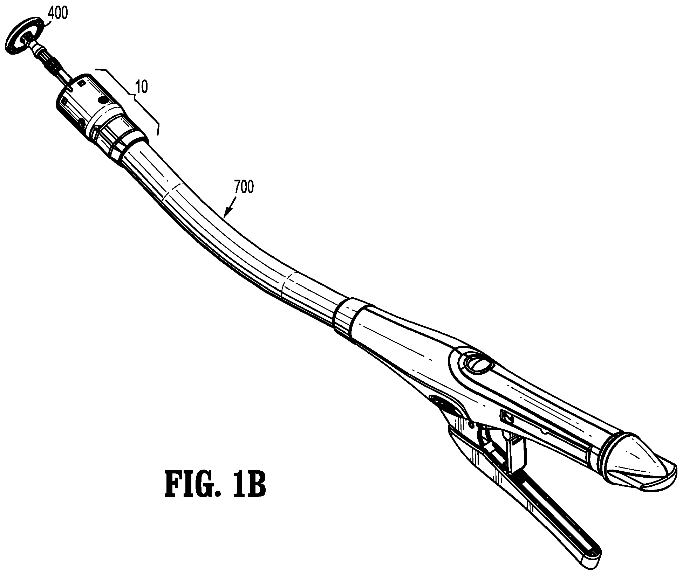

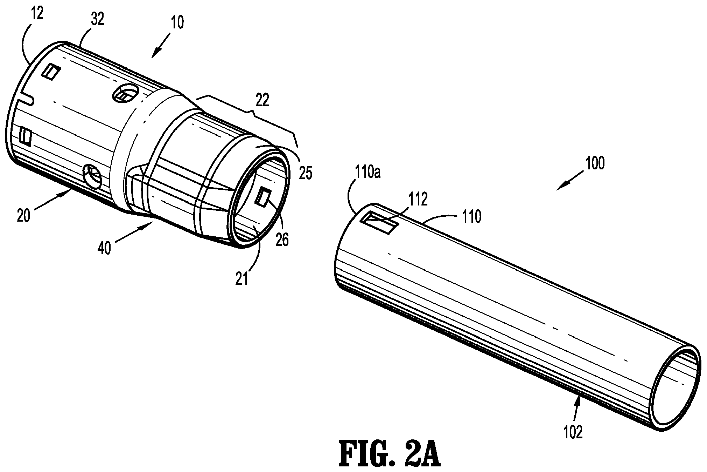

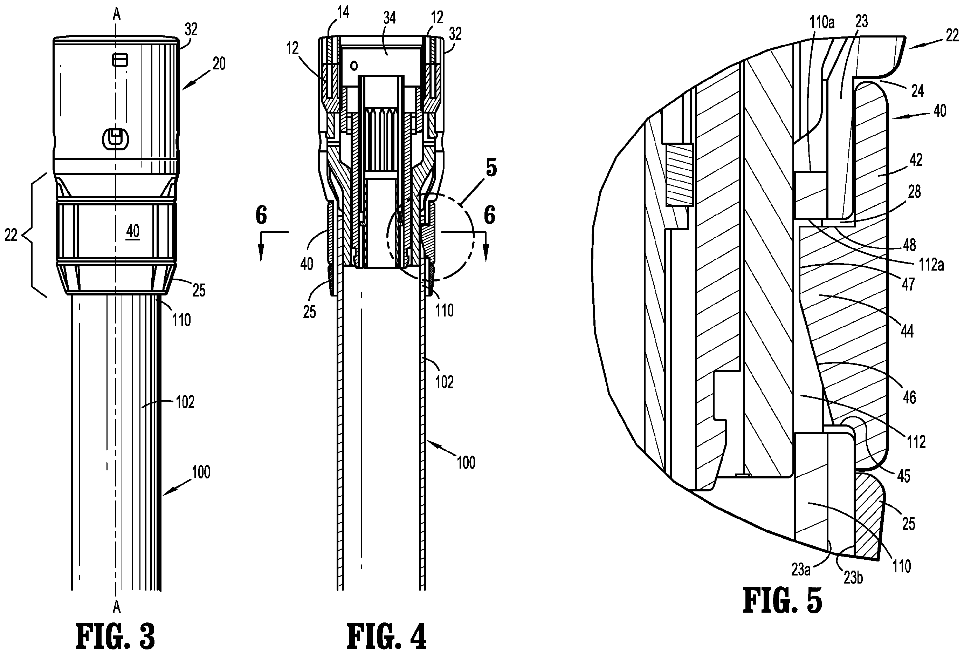

With reference to FIGS. 1A and 2A, a loading unit 10 is provided in accordance with an embodiment of the present disclosure. The loading unit 10 is configured for selective connection to a powered hand held electromechanical instrument (not shown) via an adapter 102 of a surgical instrument. Alternatively, the loading unit 10 can be configured for connection directly to a manually actuated handle assembly or stapling instrument 700 (FIG. 1B) such as described in U.S. Patent No. 8,789,737 ("the '737 Patent'). In the embodiment illustrated in FIGS. 1A and 2A, the loading unit 10 is releasably coupled to a distal end portion 110 of the adapter 102 and includes a staple cartridge 12 (FIG. 4), a shell assembly 20, and a locking collar 40. The loading unit 10 may also include an anvil 400 as shown in the illustrative embodiment of FIG. IB. The adapter 102 is configured to translate movement of a stapling instrument, e.g., an electromechanical instrument (not shown), to actuate the staple cartridge 12 to suture and cut tissue (not shown). A proximal end 104 of the adapter 102 is attachable to the stapling instrument to actuate the staple cartridge 12. It is contemplated that the proximal end 104 of the adapter 102 may be attached to a manually actuated instrument such as described in the '737 Patent to actuate the staple cartridge 12.

For a detailed description of the structure and function of an exemplary adapter and loading unit, please refer to commonly owned U.S. Provisional Patent Application Serial No. 62/066,518, filed October 21, 2014, and entitled "Adapter, Extension, and Connector Assemblies for Surgical Devices." For a detailed description of the structure and function of an exemplary electromechanical instrument, please refer to U.S. Patent Publication No. 2012/0253329.

Referring to FIG. 2A, the distal end portion 110 of surgical instrument, e.g., the adapter 102, defines a window 112. The window 112 passes through the outer surface of the distal end portion 110 of the adapter 102 and is spaced-apart from a distal end 110a of the adapter 102.

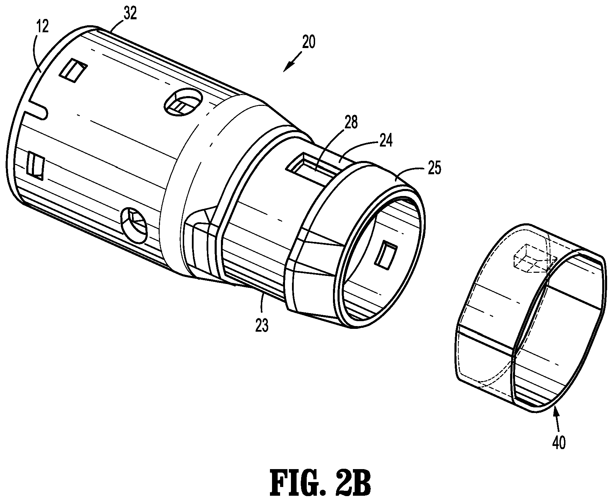

Referring also to FIG. 2B, the shell assembly 20 includes a proximal end portion 22 that defines a cylindrical opening 21 (FIG. 2A) for receiving the distal end portion 110 of the adapter 102 and a distal end 32 that defines a receptacle 34 for receiving and supporting the staple cartridge 12 (FIG. 4). The proximal end portion 22 of the shell assembly 20 includes a recessed annular ring 23 (FIG. 2B) that defines a groove 24 sized to receive the locking collar 40. In embodiments, the locking collar 40 has a thickness equal to the depth of the groove 24 such that the locking collar 40 forms a continuous or smooth surface with the outer surface of the proximal end portion 22 of the shell assembly 20. The annular ring 23 defines a lock opening 28 (FIG. 5) that receives a portion of the locking collar 40 as detailed below and defines inner and outer surfaces 23a, 23b.

The proximal end portion 22 of the shell assembly 20 supports a collar retainer 25 that retains the locking collar 40 within the groove 24. The collar retainer 25 may also be tapered to provide a smooth transition from the outer diameter of the locking collar 40 to the distal end portion 110 of the adapter 102 (or alternatively, the instrument 700 FIG. 1B). The collar retainer 25 may include locking features 26 (FIG. 2A) which secure the collar retainer 25 to the proximal end portion 22 of the shell assembly 20. It is contemplated that the collar retainer 25 may be secured to the proximal end portion 22 of the shell assembly 20 by any known means including, but not limited to, press-fitting or ultrasonic welding tabs and interlocking structure. It is also contemplated that the collar retainer 25 may be integrally formed with the proximal end portion 22 of the shell assembly 20.

With reference to FIGS. 3-6, the locking collar 40 includes a generally annular body 42 that is sized to fit within the groove 24 of the proximal end portion 22 of the shell assembly 20 and a lock 44 that extends radially inward from the annular body 42. The locking collar 40 is positioned about the proximal end portion 22 such that the lock 44 is radially aligned with the lock opening 28. The lock 44 includes a proximal step 45, a distal step 48, and an angled surface 46 positioned between the proximal and distal steps 45, 48. The proximal step 45 extends radially inward a first distance from the longitudinal axis A-A of the shell assembly 20 and the distal step 48 extends radially inward a second distance from the longitudinal axis A-A of the shell assembly 20 less than the first distance. The lock 44 may also include a landing 47 between the angled surface 46 and the distal step 48 that is substantially parallel to the longitudinal axis A-A of the shell assembly 20. The distal step 48 is positioned to engage a distal wall defining the lock opening 28 of the annular ring 23 of the shell assembly 20. The proximal step 45 is positioned adjacent a proximal wall of the lock opening 28 to longitudinally fix the locking collar 40 to the proximal end portion 22 of the shell assembly 20.

The locking collar 40 may be made of a resilient material. For example, the locking collar 12 may be formed of a resilient plastic material using an injection molding process. However, it is contemplated the locking collar 40 may be formed of other suitable materials including, but not limited to, spring steel, stainless steel, or wire.

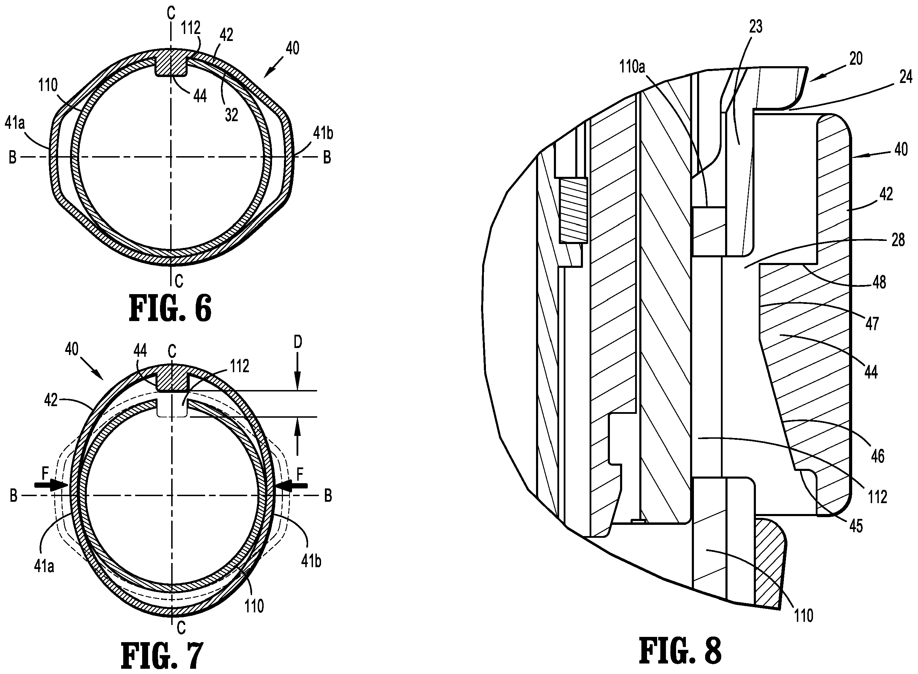

With particular reference to FIG. 6, the locking collar 40 includes first and second release surfaces 41a, 41b. Lock 44 may be positioned on the annular body 42 halfway between the first and second release surfaces 41a, 41b. In a locked configuration, the annular body 42 defines a generally elliptical shape where the annular body 42 has a width along a first axis B-B (from the first release surface 41a to the second release surface 41b) that is greater than its height along a second axis C-C (from the lock 44 to the side opposing the lock 44). The annular body 42 is biased towards the locked configuration such that the lock 44 when positioned about the annular ring 23 penetrates the inner surface 23a of the annular ring 23 (FIG. 5) and extends into the window 112 of the distal end portion 110 of the adapter 102 to secure the loading unit 10 to the surgical instrument, e.g., the adapter 102. The annular body 42 is positioned within the groove 24 about the annular ring 23 such that the distal step 48 (FIG. 5) engages a distal wall 112a (FIG. 5) defining the window 112 of the adapter 102 to longitudinally fix the shell assembly 20 to the distal end portion 110 of the adapter 102 of the surgical instrument. It will be appreciated that the lock opening 28 of the annular ring 23 of the shell assembly 20 is aligned with the window 112 of the adapter 102 in the locked configuration to permit the lock 44 to pass through the lock opening 28 and the window 112 as detailed above.

FIGS. 7 and 8 illustrate the locking collar 40 in an unlocked configuration with the annular body 42 defining a generally elliptical shape where the height is greater than the width. The release surfaces 41a, 41b can be manually pressed together to move the locking collar 40 to the unlocked configuration. In the unlocked configuration, the lock 44 is moved radially outward by distance "D" shown in FIG. 7 such that the lock 44 is positioned radially outward from the inner surface 23a of the annular ring 23 (FIG. 5). The distance "D" is greater than the distance that the lock 44 extends into the window 112 of the adapter 102 such that in the unlocked configuration, the loading unit 10 is disengaged and removable from the distal end portion 110 of the adapter 102 when the locking collar 40 is in an unlocked configuration. It is contemplated that the distance "D" may be greater than the second distance that the distal step 48 of the lock 44 extends radially inward such that in the unlocked configuration, the lock 44 is removed from the window 112 of the adapter 102 and the lock opening 28 of the proximal end portion 22 of the shell assembly 20. As shown, in the unlocked configuration, the height is greater than the width; however, it is contemplated that in the unlocked configuration the width may be greater than the height with the lock 44 moved outward by the distance "D".

Referring back to FIGS. 2-4, to couple the loading unit 10 to the surgical instrument, e.g., the adapter 102, the loading unit 10 is aligned with the adapter 102 such that the distal end portion 110 of the adapter 102 is positioned within the cylindrical opening 21 of the proximal end portion 22 with the window 112 of the adapter 102 radially aligned with the lock opening 28 of the annular ring 23 and the lock 44 of the locking collar 40. The outer surface of the locking collar 44 may include visual or tactile indicia as to the location of the lock 44. With the loading unit 10 aligned with the adapter 102, the loading unit 10 is moved proximally over the distal end portion 110 of the adapter 102 until the lock 44 is received within the window 112 of the adapter 102. It will be appreciated that the lock 44 passes through the lock opening 28 to be received within the window 112 of the adapter 102. As the loading unit 10 is moved proximally over the distal end 110 of the adapter 102, the angled surface 46 of the lock 44 engages the distal end portion 110 of the adapter 102 to transition, i.e., perform a camming action to deform, the locking collar 40, against the natural resilience of the annular body 42, from the locked configuration towards an unlocked configuration until the window 112 moves into alignment with the lock 44. When the window 112 moves into alignment with the lock 44, the lock 44 is snaps into the window 112. The snapping movement of the locking collar 40 may provide visual or audible indicia that the lock 44 is received within the window 112. It will be appreciated that the natural resilience of the annular body 42 of the locking collar 40 urges the lock 44 through the window 112 in the adapter 102. When the lock 44 is positioned within the window 112, the distal step 48 of the locking collar 40 engages the wall 112a of the adapter 102 defining the window 112 to longitudinally secure the shell assembly 20 of the loading unit 10 to the adapter 102. In addition, when the lock 44 is received within the window 112, the lock 44 prevents the loading unit 10 from rotating or twisting (i.e., radially secure) relative to the surgical instrument, e.g., the adapter 102.

With the loading unit 10 is coupled to the surgical instrument, e.g., the adapter 102, the surgical instrument and loading unit 10 may be used to perform a surgical procedure. After surgical procedure is completed, the loading unit 10 can be decoupled or detached from the surgical instrument as will be discussed in detail below. When the loading unit 10 is decoupled from the surgical instrument, another loading unit may be coupled to the surgical instrument for continued use in the surgical procedure, the surgical instrument may be sterilized for use in another surgical procedure, or the surgical instrument may be discarded. In addition, the loading unit 10 may be sterilized for use in another surgical procedure or may be discarded.

To decouple or remove the loading unit 10 from the surgical instrument, e.g., the adapter 102, the locking collar 40 is transitioned to the unlocked configuration by compressing the first and second release surfaces 41a, 41b towards one another along the first axis B-B, as represented by arrows "F" shown in FIG. 7. It is contemplated, that the locking collar 40 may be transitioned to the unlocked configuration by compressing only one of the first and second release surfaces 41a, 41b towards the other release surface 41a, 41b as shown in FIG. 7. With the locking collar 40 in the unlocked configuration, the shell assembly 20 can be removed from engagement with the distal end portion 110 of the adapter 102 by moving the shell assembly 20 axially in relation to the adapter 102.

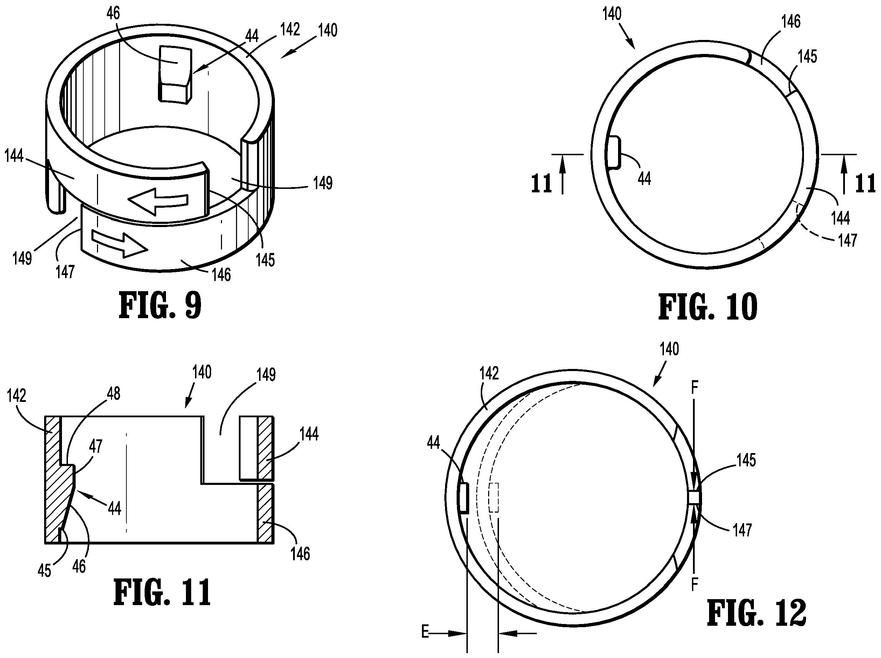

Referring now to FIGS. 9-12, another locking collar 140 is provided in accordance with the present disclosure and includes a lock 44 and an annular body 142. The lock 44 of locking collar 140 is substantially similar to the lock 44 of the locking collar 40 as detailed above and will not be discussed further below except for how it relates to locking collar 140. The annular body 142 of the locking collar 140 is similar to the annular body 42 of the locking collar 40 detailed above, as such only the differences will be detailed below for reasons of brevity.

The annular body 142 is split at one side to form first and second release surfaces or end portions 144, 146 which are moveably positioned in relation to each other such that the diameter of the locking collar 140 can be selectively changed, as described below. The first end portion144 extends in a first direction about the circumference of the annular body 142 and the second end portion 146 extends in a second direction opposite the first direction about the circumference of the annular body 142. The first and second release surfaces 144, 146 are positioned about the annular body 142 opposing the lock 44. Each of the first and second end portions 144, 146 has an end 145, 147 that defines an engagement window 149 adjacent the annular body 142. Each of the first and second end portions 144, 146 has a thickness along the longitudinal axis of the loading unit 10 (FIG. 1) approximately half the thickness of the annular body 142 such that the first and second end portions 144, 146 overlap one another about the circumference of the annular body 142.

With particular reference to FIGS. 10 and 12, the locking collar 140 is formed of a resilient material having a natural resiliency to urge the locking collar 140 towards a locked configuration (FIG. 10). In the locked configuration, the annular body 142 defines a substantially circular cross-section in a plane transverse to the longitudinal axis of the loading unit 10 (FIG. 1). In an unlocked configuration of the locking collar 140 (FIG. 12), the annular body 142 defines a generally elliptical cross-section in a plane transverse to the longitudinal axis of the loading unit 10. In the unlocked configuration, the lock 44 is moved a distance "E'" away from the first and second end portions 144, 146. The locking collar 140 is transitioned towards the unlocked configuration by urging the ends 145, 147 of the first and second end portions 144, 146, respectively, towards one another as represented by the arrows "F." As the ends 145, 147 are urged towards one another, the annular body 142 moves the lock 44 the distance "E" away from the ends 145, 147. The first and second end portions 144, 146 may include visual or tactile indicia in the form of arrows (FIG. 9) as to the direction to urge the first and second end portions 144, 146 to move the locking collar 140 towards the unlocked configuration.

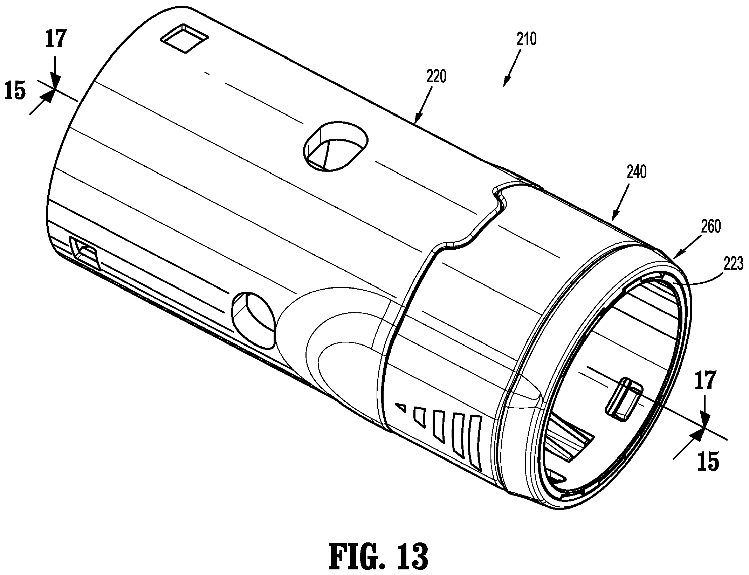

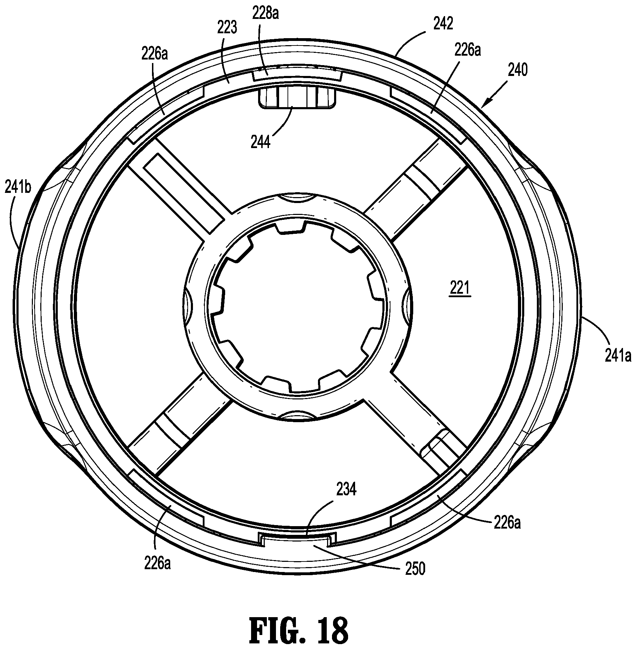

With reference to FIGS. 13-19, another loading unit 210 is provided in accordance with the present disclosure. As detailed below, the loading unit 210 includes retention tabs 252, 254 that are received within retention slots 238, 264 of a shell assembly 220 to prevent one side of a locking collar 240 from moving outward of the shell assembly 220 when release surfaces 241a, 241b of the locking collar 240 are compressed or squeezed. By limiting outward movement of one side of the locking collar 240, outward movement of the opposite side of the locking collar 240 is amplified to reduce the extent of inward movement of the release surfaces 241a, 241b necessary to release the loading unit 210 from a distal end of an instrument as discussed in detail below. The loading unit 210 includes the shell assembly 220, the locking collar 240, and the retention ring 260. The shell assembly 220 is similar to the shell assembly 20 detailed above. As such, discussion of similar features will be limited for reasons of brevity.

Referring now to FIGS. 13-14B, the shell assembly 220 includes a proximally extending annular ring 223. The annular ring 223 defines retention openings 226, a lock opening 228, relief grooves 232, and a retention groove 234. The retention openings 226 are spaced about the annular ring 223. It is contemplated that the retention openings 226 may be equally or unequally spaced about the annular ring 223. The relief grooves 232 are defined about the annular ring 223 such that the relief grooves 232 diametrically oppose one another. The lock opening 228 may be positioned halfway between the relief grooves 228. The relief grooves 232 may be flats such that the curvature of the annular ring 223 flattens to form the relief grooves 232. The retention groove 234 is positioned between relief grooves 232 opposite the lock opening 228. The retention groove 234 extends in a direction parallel to the longitudinal axis of the shell assembly 220 and may be positioned halfway between the relief grooves 232. The shell assembly 220 includes a proximally extending retention cover 238 that extends partially over the retention groove 234 to define a retention slot 236.

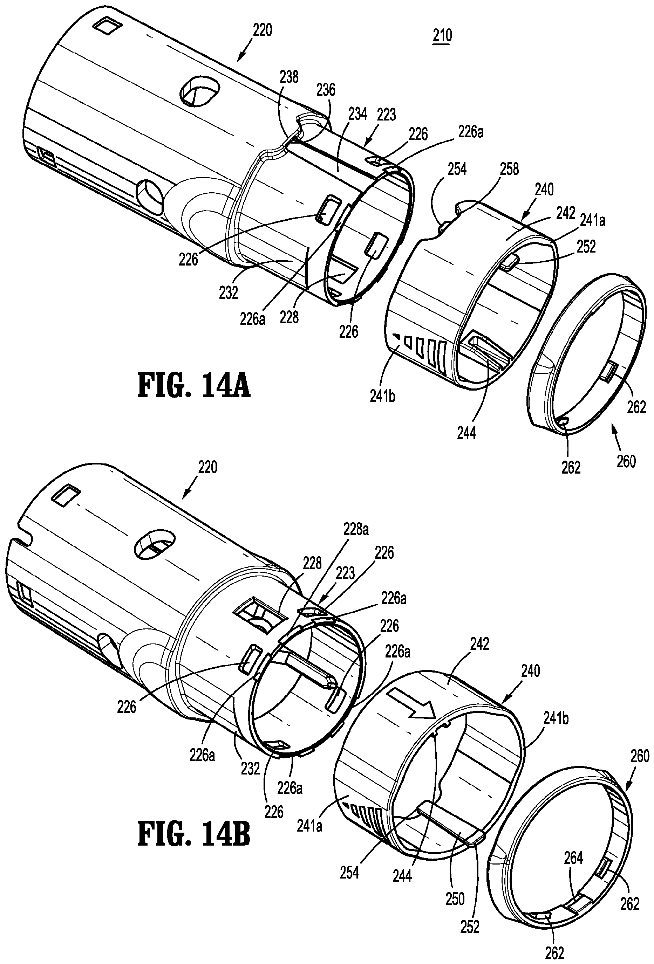

The locking collar 240 includes release surfaces 241a, 241b, a generally annular body 242, a lock 244, and a retention rib 250 (FIG. 14B). The release surfaces 241a, 241b, the body 242, and the lock 244 are similar to the release surfaces 41a, 41b, body 42, and the lock 44, respectively, detailed above with regard to locking collar 40 and will not be described herein below for reasons of brevity. The annular body 242 is sized to slidably receive the annular ring 223 of the shell assembly 220. The retention rib 250 extends inwardly from an inner surface of the body 242 between the release surfaces 241a, 241b and opposing the lock 244. The retention rib 250 includes proximal and distal retention tabs 252, 254 extending from the body 242. In embodiments, the distal side of the body 242 defines a retention cutout 258 (FIG. 14A) that is sized to receive the retention cover 238 of the shell assembly 220. As described in greater detail below, the retention rib 250 is received within the retention groove 234 to rotationally align or index the locking collar 240 with the shell assembly 220.

The retention ring 260 is positioned over the annular ring 223 of the shell assembly 220 at a position proximal to the locking collar 240 to retain the locking collar 240 over the annular ring 223 of the shell assembly 220. The retaining ring 260 includes locking tabs 262 and defines a retaining slot 264 (FIG. 14B). The locking tabs 262 extend from the inner surface of the retention ring 260 and are sized to be received within respective retention openings 226 of the annular ring 223. Each of the locking tabs 262 defines a distally facing wedge that is configured to flex the retention ring 260 outward as the retention ring 260 is slid over the annular ring 223 until the locking tabs 262 are aligned with the retention openings 226. When the locking tabs 262 are aligned with the retention openings 226, the locking tabs 262 snap through the retention openings 226 to fix the retention ring 260 to the annular ring 223. The locking tabs 262 of the retention ring 260 and the retention openings 226 of the annular ring 223 are positioned about the annular ring 223 such that when the locking tabs 262 are received within the retention openings 226, the retention slot 264 is rotationally aligned with the retention groove 234 of the annular ring 223. The annular ring 223 may define chamfers 226a at a distal end of the annular ring 223 to assist in rotationally aligning the retention ring 260 with the annular ring 223 and to assist in sliding the retention ring 260 over the annular ring 223.

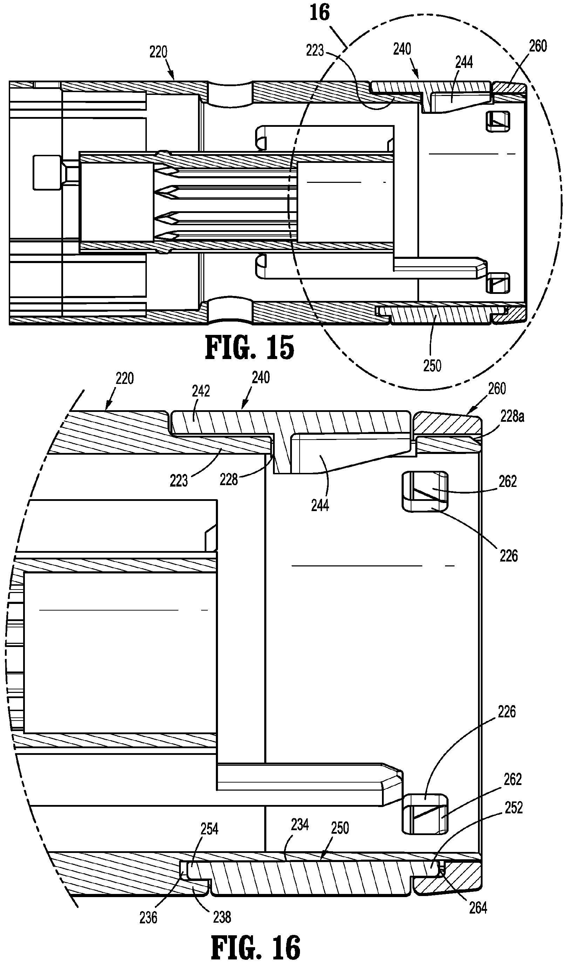

Referring also to FIGS. 15-17, a method of assembling the loading unit 210 is disclosed in accordance with the present disclosure. Initially referring to FIGS. 14A and 14B, the locking collar 240 is aligned with the shell assembly 220 such that the retention rib 250 of the locking collar 240 is aligned with the retention groove 234 defined in the annular ring 223 of the shell assembly 220. When the locking collar 240 is aligned with the shell assembly 220, the locking collar 240 is slid distally over the annular ring 223 with the retention rib 250 sliding within the retention groove 234. The locking collar 240 is slid distally over the annular ring 223 until the lock 244 of the locking collar 240 is disposed within the lock opening 228 of the annular ring 223. The annular ring 223 may define a lock chamfer 228a (FIG. 16) axially aligned with the lock opening 228 to assist in rotationally aligning the locking collar 240 with the annular ring 223 and to assist in sliding the locking collar 240 over the annular ring 223. As the locking collar 240 is slid distally over the annular ring 223, the lock 244 will abut the annular ring 223. To facilitate placement of the locking collar 240 on the annular ring 223, the release surfaces 241a, 241b can be pressed towards one another as the locking collar 240 is slid over the annular ring 223 to flex the body 242 of the locking collar 240 to move the lock 244 outward beyond the annular ring 223. When the lock 244 is aligned with the lock opening 228, the release surfaces 241a, 241b are released such that the lock 244 snaps through the lock opening 288 as shown in FIG. 16.

With particular reference to FIG. 16, as the locking collar 240 is slid over the annular ring 223, the retention rib 250 is received within the retention groove 234 of the annular ring 223 until the distal retention tab 254 is received within the retention slot 236 of the shell assembly 220. With the locking collar 240 positioned about the annular ring 223, the retention ring 260 is slid distally over the annular ring 223 to secure the locking collar 240 on the annular ring 223. In order to slide the retention ring 260 about the annular ring 223, the retention ring 260 is radially aligned with the annular ring 223 and the locking collar 240 such that the retention slot 264 of the retention ring 260 is aligned with the proximal retention tab 252 of the retention rib 250 and the retention groove 234 of the shell assembly 220. When the retention ring 260 is positioned over the annular ring 223, the locking tabs 262 snap through the retention openings 226 of the annular ring 223 to fix the retention ring 260 to a proximal end of the annular ring 223.

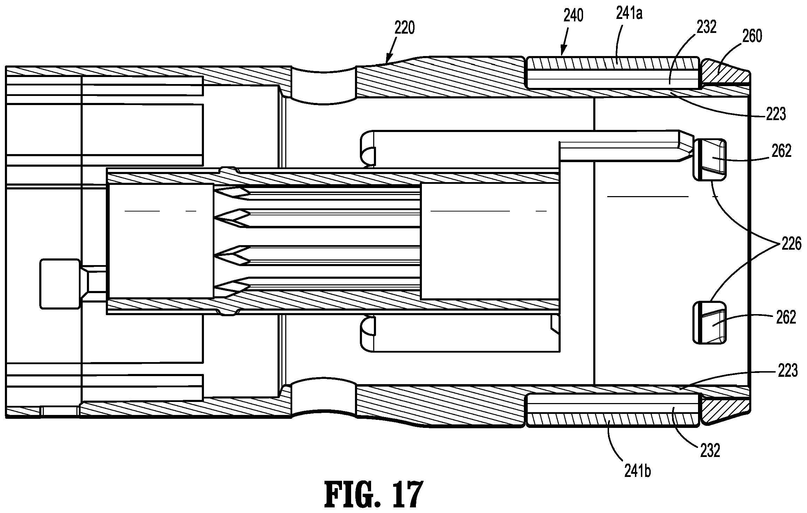

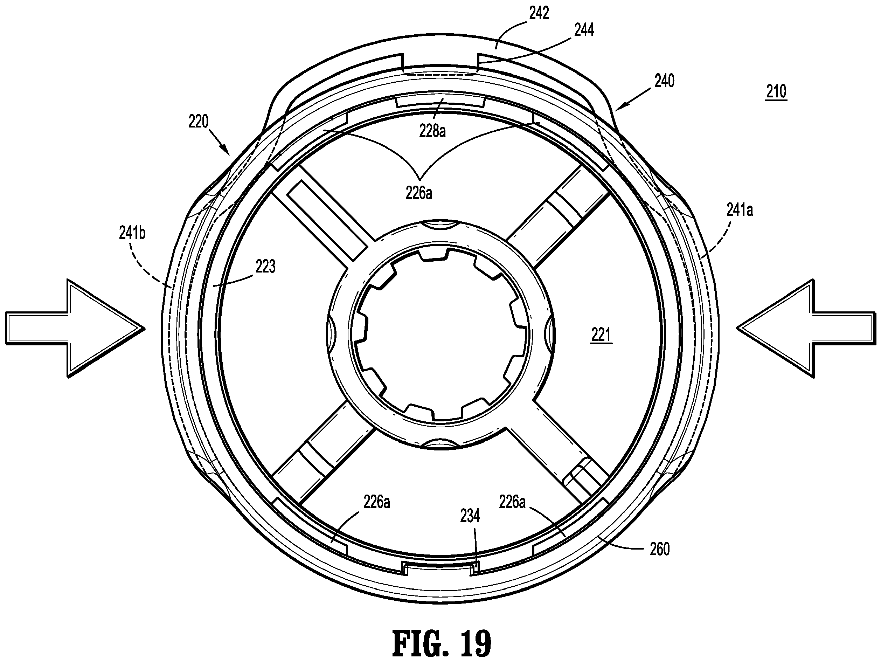

Referring to FIGS. 17-19, the relief grooves 232 of the annular ring 223 provide clearance under the release surfaces 241a, 241b of the body 242 of the locking collar 240 to facilitate inward movement of the release surfaces 241a, 241b as detailed below. When the release surfaces 241a, 241b are pressed inwardly as shown in FIG. 19, the retention tabs 252, 254 (FIG. 16) of the locking collar 240 prevent the side of the body 242 of the locking collar 240 opposite the lock 244 from moving outwardly. As such, when the release surfaces 241a, 241b are pressed inwardly, only the side of the locking collar 240 supporting the lock 244 moves outwardly. By retaining or preventing the side of the body 242 opposite the lock 244 from moving outwardly, the extent of inward movement of the releases surfaces 241a, 241b required to displace or move the lock 244 out of the lock opening 228 is reduced when compared to locking collar 40, detailed above.

As shown in FIG. 19, when both release surfaces 241a, 241b are moved inwardly, the lock 244 is moved outwardly such that the lock 244 is positioned outside of a cylindrical opening 221 defined by the annular ring 223 and beyond an outer surface of the annular ring 223. This allows the loading unit 210 to be detachable or slidable off a distal end of an adapter or surgical instrument as detailed above. It is contemplated that the relief grooves 232 can be configured such that it is necessary to move only one of the release surfaces 241a, 241b inwardly to move the lock 244 outwardly to allow detachment of the loading unit 210 from a distal end of an adapter or surgical instrument as detailed above.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned and is within the scope of the appended claims. The present disclosure is not limited to circular stapling loading units, but has application to loading units for linear stapling or other types of instruments, such as electrocautery or ultrasonic instruments. Therefore, the above description should not be construed as limiting, but merely as exemplifications of particular embodiments. Those skilled in the art will envision other modifications within the scope of the claims appended hereto.

## Claims

1. A loading unit (210) and locking collar assembly, the assembly comprising:
a shell assembly (220) having a proximally extending annular ring (223) that defines a cylindrical opening (221), the annular ring defining a lock opening (228) therethrough, the shell assembly defining a first retention slot (236) adjacent the annular ring on an opposite side of the annular ring from the lock opening;
a locking collar (240) positioned about the annular ring, the locking collar having a body (242), a lock (244), and a first retention tab (254), **characterised in that** the body is resilient and includes two release surfaces (241a, 241b) defined on the outer surface of the body (242) and being positioned on opposite sides of the body, the lock (244) extending from an inner surface of the body spaced from the two release surfaces, wherein the resilience of the body of the locking collar urges the collar to a locked configuration wherein the lock (244) extends through the lock opening (228) into the cylindrical opening (221) and wherein pressing at least one of the opposing release surfaces (241a, 241b) inwardly towards the annular ring (223) transitions the locking collar (240) to an unlocked configuration wherein the lock (244) is disposed outwardly of the cylindrical opening (241), the first retention tab (254) being received within the first retention slot (236) to prevent a side of the locking collar opposite the lock from moving outwardly in relation to the annular ring.

2. The assembly according to claim 1, wherein the annular ring defines a relief groove (232) adjacent each of the two release surfaces (241a, 241b), the relief grooves allowing inward movement of the body (242) of the locking collar (240) in the area of the release surfaces.

3. The assembly according to claim 1, further comprising a retention ring (260) positioned about the annular ring (223) proximal of the locking collar, the retention ring defining a second retention slot (264).

4. The assembly according to claim 3 wherein the locking collar (240) includes a second retention tab (252) received within the second retention slot (264) to prevent the side of the locking collar opposite the lock (244) from moving outwardly when the at least one release surface (241a, 241b) is pressed inwardly towards the annular ring (223).

5. The assembly according to claim 4, wherein the first and second retention tabs are axially aligned with one another.

6. The assembly according to claim 4 or 5, wherein the annular ring (223) defines a retention groove (234) that extends in a direction parallel to a longitudinal axis of the shell assembly, and wherein the first and second retention tabs (254, 252) extend from opposite ends of a retention rib (250) that extends inwardly from an inner surface of the body, the retention rib being disposed in the retention groove (234).

7. The assembly according to claim 6, wherein the shell assembly (220) includes a cover (238) extending distally over the retention groove (234) to define the first retention slot (236); preferably wherein the body (242) of the locking collar (240) defines a cutout (258) positioned adjacent the first retention tab (254), the cutout being sized to receive the cover of the shell assembly.

8. The assembly according to claim 3, wherein the annular ring (223) defines retention openings (226), and wherein the retention ring (260) includes locking tabs (262) extending from an inner surface thereof, each of the locking tabs being received in a respective one of the retention openings to fix the retention ring to the annular ring.

9. The assembly according to claim 8, wherein the retention openings (226) are unequally spaced about the annular ring; and/or wherein the locking tabs (262) define a wedge shape with an angled surface of the wedge facing distally; and/or wherein a proximal end of the annular ring (223) defines chamfers (226a), each of the chamfers being axially aligned with one of the retention openings.

10. The assembly according to any preceding claim, wherein a proximal end of the annular ring (223) defines a chamfer axially aligned with the lock opening.

11. A surgical system (100) comprising:
a surgical instrument including a distal end defining a lock window (112);
and a loading unit and locking collar assembly according to any one of the preceding claims; wherein
the distal end of the surgical instrument is received within the cylindrical opening (221) with the lock opening (228) radially aligned with the lock window (112); and
wherein, in the locked configuration, the lock (244) extends through into the lock window (112) to secure the shell assembly (220) to the distal end of the surgical instrument and in the unlocked configuration the lock (244) is disengaged from the lock window (112) such that the shell assembly is detachable from the distal end of the surgical instrument.

12. A method of using a loading unit and locking collar assembly according to any one of claims 1 to 10, the method comprising:
aligning the locking collar (240) with the shell assembly (220); and
sliding the loading unit (210) distally over a proximally extending annular ring (223) of the shell assembly, the lock (244) of the locking collar engaging the annular ring such that the lock is moved outwardly towards an unlocked configuration of the locking collar until the lock is aligned with the lock opening (228) defined through the annular ring, resilience of a body of the locking collar urging the locking collar towards the locked configuration of the locking collar to move the lock through the lock opening, the retention tab (254) of the locking collar positioned on an opposite side of the locking collar from the lock sliding into a retention slot defined by the shell assembly to prevent the opposite side of the locking collar from moving outwardly.

13. The method according to claim 12, further comprising attaching the loading unit to a distal end of a surgical instrument (100) including:
aligning the loading unit (210) with the distal end of the surgical instrument; and
sliding the loading unit over the distal end of the surgical instrument such that the lock (244) of the locking collar engages the distal end of the surgical instrument, the distal end moving the lock outwardly to urge the locking collar towards the unlocked configuration until the lock is aligned with a locking window (112) defined in the distal end of the surgical instrument, the lock being urged through the locking window by resilience of the body of the locking collar to secure the loading unit to the distal end of the surgical instrument.

14. The method according to claim 13, further comprising actuating a shell assembly (220) of the loading unit with the surgical instrument after the loading unit is attached to the distal end of the surgical instrument.

15. The method according to claim 14, further comprising detaching the loading unit from the distal end of the surgical instrument after actuating the shell assembly including:
moving at least one of the release surfaces (141a, 241b) of the locking collar inwardly to move the lock (244) outwardly such that the lock is disengaged from the locking window of the distal end of the surgical instrument, the retention tab of the locking collar engaging the shell assembly to prevent the side of the locking collar opposite the lock from moving outwardly in response to moving the release surface of the locking collar inwardly; and
sliding the loading unit off of the distal end of the surgical instrument.

## Patentansprüche

1. Ladeeinheit- (210) und Verriegelungsringanordnung, wobei die Anordnung umfasst:
eine Hülsenanordnung (220) mit einem sich proximal erstreckenden ringförmigen Ring (223), der eine zylindrische Öffnung (221) definiert, wobei der ringförmige Ring eine Verriegelungsöffnung (228) dort hindurch definiert, wobei die Hülsenanordnung einen ersten Aufbewahrungsschlitz (236) benachbart zu dem ringförmigen Ring auf einer gegenüberliegenden Seite des ringförmigen Rings von der Verriegelungsöffnung definiert;
einen Verriegelungsring (240), der um den ringförmigen Ring herum positioniert ist, wobei der Verriegelungsring einen Körper (242), eine Verriegelung (244) und eine erste Aufbewahrungslasche (254) aufweist, **dadurch gekennzeichnet, dass** der Körper elastisch ist und zwei Freigabeflächen (241a, 241b) aufweist, die auf der Außenfläche des Körpers (242) definiert und auf gegenüberliegenden Seiten des Körpers positioniert sind, wobei sich die Verriegelung (244) von einer Innenfläche des Körpers erstreckt, die von den zwei Freigabeflächen beabstandet ist, wobei die Elastizität des Körpers des Verriegelungsrings den Ring in eine verriegelte Konfiguration drängt, bei der sich die Verriegelung (244) durch die Verriegelungsöffnung (228) in die zylindrische Öffnung (221) erstreckt, und wobei Drücken zumindest einer der gegenüberliegenden Freigabeflächen (241a, 241b) nach innen in Richtung des ringförmigen Rings (223) den Verriegelungsring (240) in eine entriegelte Konfiguration überführt, bei der die Verriegelung (244) außerhalb der zylindrischen Öffnung (241) angeordnet ist, wobei die erste Aufbewahrungslasche (254) innerhalb des ersten Aufbewahrungsschlitzes (236) aufgenommen ist, um zu verhindern, dass sich eine Seite des Verriegelungsrings gegenüber der Verriegelung in Bezug auf den ringförmigen Ring nach außen bewegt.

2. Anordnung nach Anspruch 1, wobei der ringförmige Ring eine Entlastungsrille (232) benachbart zu jeder der zwei Freigabeflächen (241a, 241b) definiert, wobei die Entlastungsrillen eine Einwärtsbewegung des Körpers (242) des Verriegelungsrings (240) im Bereich der Freigabeflächen erlauben.

3. Anordnung nach Anspruch 1, weiter umfassend einen Aufbewahrungsring (260), der um den ringförmigen Ring (223) proximal des Verriegelungsrings positioniert ist, wobei der Aufbewahrungsring einen zweiten Aufbewahrungsschlitz (264) definiert.

4. Anordnung nach Anspruch 3, wobei der Verriegelungsring (240) eine zweite Aufbewahrungslasche (252) aufweist, die innerhalb des zweiten Aufbewahrungsschlitzes (264) aufgenommen ist, um zu verhindern, dass sich die Seite des Verriegelungsrings gegenüber der Verriegelung (244) nach außen bewegt, wenn die zumindest eine Freigabefläche (241a, 241b) nach innen in Richtung des ringförmigen Rings (223) gedrückt wird.

5. Anordnung nach Anspruch 4, wobei die ersten und zweiten Aufbewahrungslaschen axial zueinander ausgerichtet sind.

6. Anordnung nach Anspruch 4 oder 5, wobei der ringförmige Ring (223) eine Aufbewahrungsrille (234) definiert, die sich in einer Richtung parallel zu einer Längsachse der Hülsenanordnung erstreckt, und wobei sich die ersten und zweiten Aufbewahrungslaschen (254, 252) von gegenüberliegenden Enden einer Aufbewahrungsrippe (250) erstrecken, die sich von einer Innenfläche des Körpers nach innen erstreckt, wobei die Aufbewahrungsrippe in der Aufbewahrungsrille (234) angeordnet ist.

7. Anordnung nach Anspruch 6, wobei die Hülsenanordnung (220) eine Abdeckung (238) aufweist, die sich distal über die Aufbewahrungsrille (234) erstreckt, um den ersten Aufbewahrungsschlitz (236) zu definieren; wobei der Körper (242) des Verriegelungsrings (240) vorzugsweise einen Ausschnitt (258) definiert, der benachbart zu der ersten Aufbewahrungslasche (254) positioniert ist, wobei der Ausschnitt so bemessen ist, dass er die Abdeckung der Hülsenanordnung aufnimmt.

8. Anordnung nach Anspruch 3, wobei der ringförmige Ring (223) Aufbewahrungsöffnungen (226) definiert und wobei der Aufbewahrungsring (260) Verriegelungslaschen (262) aufweist, die sich von einer Innenfläche davon erstrecken, wobei jede der Verriegelungslaschen in einer entsprechenden der Aufbewahrungsöffnungen aufgenommen wird, um den Aufbewahrungsring an dem ringförmigen Ring zu befestigen.

9. Anordnung nach Anspruch 8, wobei die Aufbewahrungsöffnungen (226) ungleichmäßig um den ringförmigen Ring herum beabstandet sind; und/oder wobei die Verriegelungslaschen (262) eine Keilform mit einer distal gerichteten abgewinkelten Fläche des Keils definieren; und/oder wobei ein proximales Ende des ringförmigen Rings (223) Fasen (226a) definiert, wobei jede der Fasen mit einer der Aufbewahrungsöffnungen axial ausgerichtet ist.

10. Anordnung nach einem vorstehenden Anspruch, wobei ein proximales Ende des ringförmigen Rings (223) eine Fase definiert, die mit der Verriegelungsöffnung axial ausgerichtet ist.

11. Chirurgisches System (100) umfassend:
ein chirurgisches Instrument, das ein distales Ende aufweist, das ein Verriegelungsfenster (112) definiert;
und eine Ladeeinheit- und Verriegelungsringanordnung nach einem der vorstehenden Ansprüche; wobei
das distale Ende des chirurgischen Instruments innerhalb der zylindrischen Öffnung (221) aufgenommen ist, wobei die Verriegelungsöffnung (228) mit dem Verriegelungsfenster (112) radial ausgerichtet ist; und
wobei sich in der verriegelten Konfiguration die Verriegelung (244) durch in das Verriegelungsfenster (112) erstreckt, um die Hülsenanordnung (220) an dem distalen Ende des chirurgischen Instruments zu sichern, und in der entriegelten Konfiguration die Verriegelung (244) von dem Verriegelungsfenster (112) gelöst ist, so dass die Hülsenanordnung von dem distalen Ende des chirurgischen Instruments getrennt werden kann.

12. Verfahren zur Verwendung einer Ladeeinheit- und Verriegelungsringanordnung nach einem der Ansprüche 1 bis 10, wobei das Verfahren umfasst:
Ausrichten des Verriegelungsrings (240) mit der Hülsenanordnung (220); und
Schieben der Ladeeinheit (210) distal über einen sich proximal erstreckenden ringförmigen Ring (223) der Hülsenanordnung, wobei die Verriegelung (244) des Verriegelungsrings mit dem ringförmigen Ring in Eingriff steht, so dass die Verriegelung nach außen in Richtung einer entriegelten Konfiguration des Verriegelungsrings bewegt wird, bis die Verriegelung mit der Verriegelungsöffnung (228) ausgerichtet ist, die durch den ringförmigen Ring definiert ist, wobei die Elastizität eines Körpers des Verriegelungsrings den Verriegelungsring in Richtung der verriegelten Konfiguration des Verriegelungsrings drängt, um die Verriegelung durch die Verriegelungsöffnung zu bewegen, wobei die Aufbewahrungslasche (254) des Verriegelungsrings auf einer gegenüberliegenden Seite des Verriegelungsrings von der Verriegelung positioniert ist, die in einen Aufbewahrungsschlitz gleitet, der durch die Hülsenanordnung definiert ist, um zu verhindern, dass sich die gegenüberliegende Seite des Verriegelungsrings nach außen bewegt.

13. Verfahren nach Anspruch 12, weiter umfassend Befestigen der Ladeeinheit an einem distalen Ende eines chirurgischen Instruments (100) aufweisend:
Ausrichten der Ladeeinheit (210) mit dem distalen Ende des chirurgischen Instruments; und
Schieben der Ladeeinheit über das distale Ende des chirurgischen Instruments, so dass die Verriegelung (244) des Verriegelungsrings mit dem distalen Ende des chirurgischen Instruments in Eingriff steht, wobei das distale Ende die Verriegelung nach außen bewegt, um den Verriegelungsring in Richtung der entriegelten Konfiguration zu drängen, bis die Verriegelung mit einem Verriegelungsfenster (112) ausgerichtet ist, das in dem distalen Ende des chirurgischen Instruments definiert ist, wobei die Verriegelung durch die Elastizität des Körpers des Verriegelungsrings durch das Verriegelungsfenster gedrängt wird, um die Ladeeinheit an dem distalen Ende des chirurgischen Instruments zu sichern.

14. Verfahren nach Anspruch 13, weiter umfassend Betätigen einer Hülsenanordnung (220) der Ladeeinheit mit dem chirurgischen Instrument nachdem die Ladeeinheit an dem distalen Ende des chirurgischen Instruments befestigt ist.

15. Verfahren nach Anspruch 14, weiter umfassend Trennen der Ladeeinheit von dem distalen Ende des chirurgischen Instruments nach Betätigung der Hülsenanordnung aufweisend:
Bewegen zumindest einer der Freigabeflächen (141a, 241b) des Verriegelungsrings nach innen, um die Verriegelung (244) nach außen zu bewegen, so dass die Verriegelung von dem Verriegelungsfenster des distalen Endes des chirurgischen Instruments gelöst wird, wobei die Aufbewahrungslasche des Verriegelungsrings mit der Hülsenanordnung in Eingriff steht, um zu verhindern, dass sich die Seite des Verriegelungsrings gegenüber der Verriegelung nach außen bewegt, als Reaktion auf eine Bewegung der Freigabefläche des Verriegelungsrings nach innen; und
Schieben der Ladeeinheit vom distalen Ende des chirurgischen Instruments.

## Revendications

1. Unité de chargement (210) et ensemble de collier de verrouillage, l'ensemble comprenant :
un ensemble de coque (220) ayant une bague annulaire (223) s'étendant de manière proximale qui définit une ouverture cylindrique (221), la bague annulaire définissant une ouverture de verrouillage (228) à travers celle-ci, l'ensemble de coque définissant une première fente de rétention (236) adjacente à la bague annulaire sur un côté opposé de la bague annulaire à partir de l'ouverture de verrouillage ;
un collier de verrouillage (240) positionné autour de la bague annulaire, le collier de verrouillage ayant un corps (242), un verrou (244), et une première patte de rétention (254), **caractérisé en ce que** le corps est élastique et comprend deux surfaces de libération (241a, 241b) définies sur la surface extérieure du corps (242) et étant positionnés sur des côtés opposés du corps, le verrou (244) s'étendant à partir d'une surface intérieure du corps espacée des deux surfaces de libération, dans lequel l'élasticité du corps du collier de verrouillage presse le collier dans une configuration verrouillée dans laquelle le verrou (244) s'étend à travers l'ouverture de verrouillage (228) dans l'ouverture cylindrique (221) et dans lequel on presse l'une au moins des surfaces de libération opposées (241a, 241b) vers l'intérieur vers la bague annulaire (223), le collier de verrouillage (240) transite dans une configuration déverrouillée dans laquelle le verrou (244) est disposé à l'extérieur de l'ouverture cylindrique (241), la première patte de rétention (254) étant reçue dans la première fente de rétention (236) pour éviter qu'un côté du collier de verrouillage opposée au verrou se déplace vers l'extérieur par rapport à la bague annulaire.

2. Ensemble selon la revendication 1, dans lequel la bague annulaire définit une rainure en relief (232) adjacente à chacune des deux surfaces de libération (241a, 241b), les rainures en relief permettant un mouvement vers l'intérieur du corps (242) du collier de verrouillage (240) dans la zone des surfaces de libération.

3. Ensemble selon la revendication 1, comprenant en outre une bague de rétention (260) positionnée autour de la bague annulaire (223) à proximité du collier de verrouillage, la bague de rétention définissant une seconde fente de rétention (264).

4. Ensemble selon la revendication 3 dans lequel le collier de verrouillage (240) comprend une deuxième patte de rétention (252) reçue dans la deuxième fente de rétention (264) pour éviter le côté du collier de verrouillage opposé au verrou (244) de se déplacer vers l'extérieur lorsque la au moins une surface de libération (241a, 241b) est pressée vers l'intérieur vers de la bague annulaire (233).

5. Ensemble selon la revendication 4, dans lequel les première et seconde pattes de rétention sont axialement alignées l'une avec l'autre.

6. Ensemble selon la revendication 4 ou 5, dans lequel la bague annulaire (223) définit une rainure de rétention (234) qui s'étend dans une direction parallèle à un axe longitudinal de l'ensemble de coque, et dans lequel les première et seconde pattes de rétention (254, 252) s'étendent depuis les extrémités opposées d'une nervure de rétention (250) qui s'étend vers l'intérieur depuis une surface intérieure du corps, la nervure de rétention étant disposée dans la rainure de rétention (234).

7. Ensemble selon la revendication 6, dans lequel l'ensemble de coque (220) comprend un couvercle (238) s'étendant de manière distale sur la rainure de rétention (234) pour définir la première fente de rétention (236) ; de préférence dans lequel le corps (242) du collier de verrouillage (240) définit une découpe (258) positionnée de manière adjacente à la première patte de rétention (254), la découpe étant dimensionnée pour recevoir le couvercle de l'ensemble de coque.

8. Ensemble selon la revendication 3, dans lequel la bague annulaire (223) définit des ouvertures de rétention (226), et dans lequel la bague de rétention (260) comprend des pattes de verrouillage (262) s'étendant depuis une surface intérieure de celle-ci, chacune des pattes de verrouillage étant reçue dans une des ouvertures de rétention respectives pour fixer la bague de rétention à la bague annulaire.

9. Ensemble selon la revendication 8, dans lequel les ouvertures de rétention (226) sont espacées de manière inégale autour de la bague annulaire ; et / ou dans lequel les pattes de verrouillage (262) définissent une forme de coin avec une surface inclinée du coin faisant face de manière distale ; et / ou dans lequel une extrémité proximale de la bague annulaire (223) définit des chanfreins (226a), chacun des chanfreins étant aligné axialement avec l'une des ouvertures de rétention.

10. Ensemble selon l'une quelconque des revendications précédentes, dans lequel une extrémité proximale de la bague annulaire (223) définit un chanfrein aligné axialement avec l'ouverture de verrouillage.

11. Système chirurgical (100) comprenant :
un instrument chirurgical comprenant une extrémité distale définissant une fenêtre de verrouillage (112);
et une unité de chargement et un ensemble de collier de verrouillage selon l'une quelconque des revendications précédentes, dans lequel
l'extrémité distale de l'instrument chirurgical est reçue dans l'ouverture cylindrique (221) avec l'ouverture de verrouillage (228) radialement alignée avec la fenêtre de verrouillage (112); et dans lequel dans la configuration verrouillée, le verrou (244) s'étend à travers jusque dans la fenêtre de verrouillage (112) pour fixer l'ensemble de coque (220) à l'extrémité distale de l'instrument chirurgical et dans la configuration déverrouillée le verrou (244) est désengagé de la fenêtre de verrouillage (112) de telle sorte que l'ensemble de coque puisse être détaché de l'extrémité distale de l'instrument chirurgical.

12. Procédé d'utilisation d'un ensemble de chargement et de collier de verrouillage selon l'une quelconque des revendications 1 à 10, le procédé comprenant :
aligner un collier de verrouillage (240) avec un ensemble de coque (220); et
glisser de manière distale l'unité de chargement (210) sur une bague annulaire (223) s'étendant de manière proximale de l'ensemble de coque, le verrou (244) du collier de verrouillage s'engage avec la bague annulaire de sorte que le verrou est déplacée vers l'extérieur vers une configuration déverrouillée du collier de verrouillage jusqu'à ce que le verrou est aligné avec l'ouverture de verrouillage (228) définie à travers la bague annulaire, l'élasticité d'un corps du collier de verrouillage poussant le collier de verrouillage vers la configuration verrouillée du collier de verrouillage pour déplacer le verrou au travers de l'ouverture de verrouillage, une patte de rétention (254) du collier de verrouillage positionné sur un côté opposé du collier de verrouillage par rapport au verrou coulissant dans une fente de rétention définie par l'ensemble de coque pour éviter le côté opposé du collier de verrouillage de se déplacer vers l'extérieur.

13. Procédé selon la revendication 12, comprenant en outre la fixation de l'unité de chargement à une extrémité distale d'un instrument chirurgical (100) comprenant :
aligner l'unité de chargement (210) avec l'extrémité distale de l'instrument chirurgical; et
glisser l'unité de chargement sur l'extrémité distale de l'instrument chirurgical de sorte que le verrou (244) du collier de verrouillage s'engage dans l'extrémité distale de l'instrument chirurgical, l'extrémité distale déplaçant le verrou vers l'extérieur pour pousser le collier de verrouillage vers la configuration déverrouillée jusqu'à ce que le verrou est aligné avec une fenêtre de verrouillage (112) définie dans l'extrémité distale de l'instrument chirurgical, le verrou étant poussé à travers la fenêtre de verrouillage par élasticité du corps du collier de verrouillage pour fixer l'unité de chargement à l'extrémité distale de l'instrument chirurgical.

14. Procédé selon la revendication 13, comprenant en outre l'actionnement d'un ensemble de coque (220) de l'unité de chargement avec l'instrument chirurgical après que l'unité de chargement ait été fixée à l'extrémité distale de l'instrument chirurgical.

15. Procédé selon la revendication 14, comprenant en outre le détachement de l'unité de chargement de l'extrémité distale de l'instrument chirurgical après l'actionnement de l'ensemble de coque comprenant :
déplacer au moins l'une des surfaces de libération (141a, 241b) du collier de verrouillage vers l'intérieur pour déplacer le verrou (244) vers l'extérieur de telle sorte que le verrou est désengagé de la fenêtre de verrouillage de l'extrémité distale de l'instrument chirurgical, la patte de rétention du collier de verrouillage engageant l'ensemble de coque pour éviter le côté du collier de verrouillage opposé au verrou de se déplacer vers l'extérieur en réponse au déplacement de la surface de libération du collier de verrouillage vers l'intérieur; et
glisser l'unité de chargement hors de l'extrémité distale de l'instrument chirurgical.
